(19) **Europäisches Patentamt**
**European Patent Office**
**Office européen des brevets**

(11) **EP 2 685 889 B1**

(12) **EUROPEAN PATENT SPECIFICATION**

(45) Date of publication and mention
of the grant of the patent:
**14.08.2019 Bulletin 2019/33**

(21) Application number: **12712365.1**

(22) Date of filing: **09.03.2012**

(51) Int Cl.:
***A61B 5/08*** (2006.01)

(86) International application number:
**PCT/IB2012/051121**

(87) International publication number:
**WO 2012/123874 (20.09.2012 Gazette 2012/38)**

(54) **BREATHLESSNESS AND EDEMA SYMPTOM ASSESSMENT**

BEURTEILUNG VON ATEMLOSIGKEITS- UND ÖDEM-SYMPTOMEN

ÉVALUATION DE SYMPTÔME D'OEDÈME ET D'ESSOUFFLEMENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.03.2011 US 201161453153 P**

(43) Date of publication of application:
**22.01.2014 Bulletin 2014/04**

(73) Proprietor: **Koninklijke Philips N.V.
5656 AE Eindhoven (NL)**

(72) Inventors:
• **IVANOVIC, Ana
5656 AE Eindhoven (NL)**

• **TESANOVIC, Aleksandra
5656 AE Eindhoven (NL)**
• **ROCK, Joseph, Ernest
5656 AE Eindhoven (NL)**

(74) Representative: **Steffen, Thomas
Philips Intellectual Property & Standards
High Tech Campus 5
5656 AE Eindhoven (NL)**

(56) References cited:
**WO-A1-98/33553      WO-A1-2005/036525
WO-A1-2009/090600    US-A1- 2004 210 159
US-A1- 2005 119 586   US-A1- 2005 137 481
US-A1- 2010 198 097   US-A1- 2010 298 649**

## Description

### Background

Patients suffering from chronic conditions may perform in-home monitoring of their symptoms in order to evaluate whether their condition is improving or deteriorating. Self-monitoring may enable patents' condition to be monitored at a cost significantly less than that of in-home care or inpatient treatment. However, self-monitoring also suffers from deficiencies due to subjective assessment and lack of detailed objective information provided by the patient.

WO 98/33553 A1 discloses an impedance monitor for discerning edema through evaluation of respiratory rate.

US 2004/0210159 A1 discloses a method for determining a physiological state of a human subject. A set of measurements are performed to receive information that relate to a subject's mental state. These information are analyzed together to produce a determination of the subject's emotional state or cognitive state.

US 2010/0198097 A1 discloses a device for detecting worsening heart failure based on fluid accumulation with respiratory confirmation. The device uses intrathoracic impedance measurements to monitor both the fluid accumulation and one or more respiratory parameters.

US 2005/0137481 A1 discloses a method and device for remote management of patients suffering from heart failure and hypertension.

US 2010/0298649 A1 discloses a system and method for the assessment of a brain status of a subject.

WO 2005/036525 A1 discloses a method, a system and a computer program product for speech recognition with environmental adaptation.

US 2005/0119586 A1 discloses systems and methods for processing respiratory signals derived generally from respiratory plethysmography.

### Summary of the Invention

According to a first aspect of the present invention, a system as claimed in claim 1 is presented. According to further aspects of the present invention, a method as claimed in claim 5 and a computer program product as claimed in claim 9 are presented.

The system includes a voice recording apparatus, a speech recognition unit, and a processor. The voice recording apparatus records an audio recording of a patient during a time interval. The speech recognition unit receives the audio recording and determines, from the audio recording, a number of pauses during the time interval and a length of each of the pauses. The processor determines, based on the number of pauses and the length of each of the pauses, a breathlessness score for the patient corresponding to the time interval.

The method comprises the steps of: receiving an audio recording of a patient during a time interval; determining, from the audio recording, a number of pauses during the time interval and a length of each of the pauses; determining, based on the number of pauses and the length of each of the pauses, a breathlessness score for the patient corresponding to the time interval; and providing an alert to one of the patient and a medical professional, if the determined breathlessness score is greater than an acceptable level.

### Brief Description of the Drawings

Figure 1 shows an exemplary embodiment of a system for the objective assessment of patient symptoms.

Figure 2 shows an exemplary embodiment of an ankle measurement device.

Figure 3 shows an exemplary embodiment of a method for the objective assessment of patient symptoms.

Figure 4 depicts a specific method of analyzing specific types of patient data.

Figure 5 depicts another specific method of analyzing specific types of patient data.

### Detailed Description

The exemplary embodiments of the present invention may be further understood with reference to the following description and the appended drawings, wherein like elements are referred to with the same reference numerals. The exemplary embodiments describe systems and methods by which patient symptoms may be monitored and evaluated objectively in order to determine whether a patient's condition has improved or deteriorated.

The periodic assessment of the symptoms affecting patients is crucial for the monitoring of patients suffering from chronic conditions. This is especially important for patients suffering from heart failure, but also true for patients suffering from other chronic conditions, such as chronic obstructive pulmonary disease ("COPD"), kidney failure, etc. Such monitoring is required to determine whether a patient's treatment needs to be modified (e.g., whether medication is required, whether dosing of existing medication should be changed, whether hospitalization is required, whether closer monitoring by medical professionals is required, etc.).

However, existing methods for making such decisions are typically based on information provided by patients in response to self-administered questionnaires, which prompt a patient to answer a series of questions, rate his own symptoms, etc. One type of questionnaire may ask a patient to report loss of breath during various activities (e.g., sitting, standing, walking, etc.). Another type of questionnaire may ask a patient to report on swelling of various parts of the body (e.g., ankles, legs etc.) at various times of the day (e.g., morning, midday, evening, etc.). Another type of questionnaire may ask a patient to report on general well-being (e.g., happy, neutral, unhappy). Another type of questionnaire may ask a patient to report on weight gain or weight loss. In each of these cases, patients are asked questions relating to the above conditions on a regular basis, either face-to-face or by a telemonitoring system, and the patient reports on his own conditions.

In one example, the accumulation of fluids in bodily tissues, known as edema, may be a sign of worsening heart failure, kidney damage, or COPD. Edema may be observed by observing swelling in a patient's body, typically in the ankles and legs but also in the abdomen or the hands. Thus, by observing such swelling, treatment may be administered before hospitalization is required. Patients may self-assess edema symptoms by monitoring their weight and/or swollen body parts daily.

This method of gathering information may suffer from various deficiencies. First, surveys that require patients to answer a variety of questions at periodic intervals may be difficult, cumbersome, or tedious to complete. In such cases, a patient may fail to answer questions or provide inaccurate information. Second, the information requested is subjective, and the reliability of the resulting data may therefore be suspect. For example, a patient who is self-assessing edema may not be capable of distinguishing between different levels of congestion. Third, the surveys do not necessarily result in information that is detailed enough to deduct the cause of a particular symptom, resulting in the need for medical personnel to spend time ascertaining further information (e.g., type of activity during which reported shortness of breath occurred, potential causes for reported weight gain, etc.) .

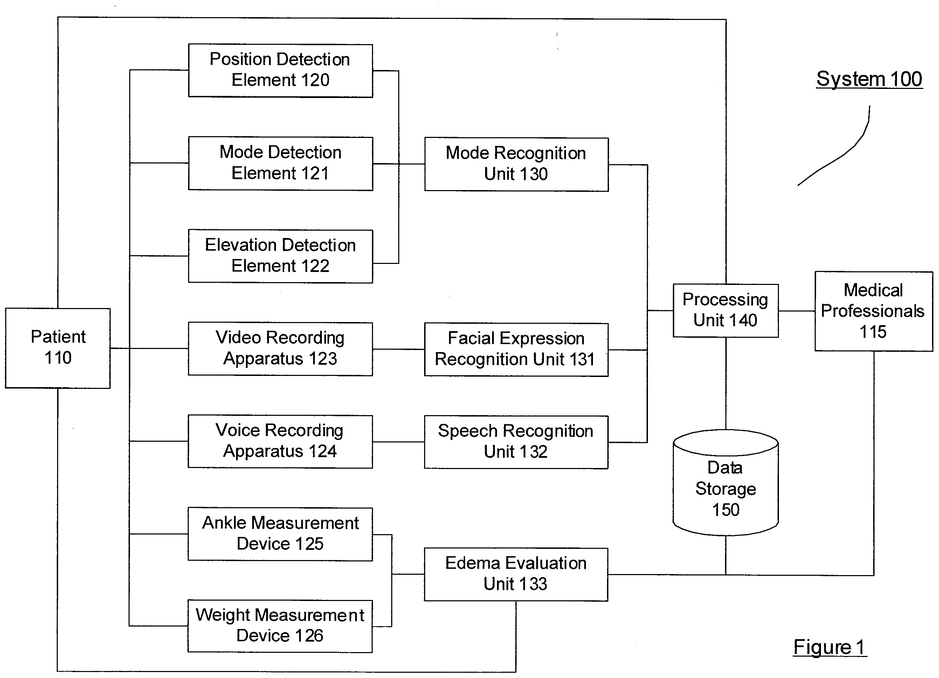

The exemplary embodiments aid in the assessment of patient symptoms by providing simplified and objective assessment of symptoms that may be assessed subjectively by prior methods. An exemplary system 100, illustrated in Figure 1, objectively measures the symptoms of a patient 10, as will be described below, in order to make determinations about the patient's condition. Feedback about these determinations may be provided to the patient 110, to medical professionals 115 (e.g., doctors, nurses, etc.), or both.

Patient symptoms are measured by one or more mechanisms, including a position detection element 120, a mode detection element 121, an elevation detection element 122, a video recording apparatus 123, a voice recording apparatus 124, an ankle measurement device 125, and a weight measurement device 126. The operation of each of these mechanisms will be described in further detail below.

The position detection element 120 detects a position of the patient 110 (e.g., laying, sitting, standing, etc.). The position may affect the significance of any breathlessness being experienced by the patient 110. For example, loss of breath experienced by a patient who is laying down may be a more significant indication of a worsening condition than loss of breath experienced by a patient who is standing. For a patient who is being monitored at home, the position detection element 120 may be a gyroscope (e.g., part of an activity monitoring device) configured to determine the patient's position using methods that are known in the art. For a patient who is being monitored in the hospital, the position detection element 120 may be a sensor mounted on the patient's hospital bed.

The mode detection element 121 detects an activity mode of the patient 110 (e.g., stationary, walking, running, etc.). As was the case for the patient's position, the mode may affect the significance of any breathlessness being experienced by the patient 110. For example, loss of breath experienced by a patient who is stationary may be a more significant indication of a worsening condition than loss of breath experienced by a patient who is moving. The mode detection element 121 may be an accelerometer (e.g., part of an activity monitoring device) configured to determine the patient's mode using methods that are known in the art. This may be, for example, as part of a laptop computer or mobile phone used by the patient. For a patient who is hospitalized, the mode detection element 121 may be a bed-mounted sensor that detects if the bed is occupied (e.g., the patient is stationary) or empty (e.g., the patient is moving), or, alternately, may be a piece of exercise equipment (e.g., a treadmill) used in physical therapy that detects the same information.

The elevation detection element 122 detects changes in the elevation of the patient 110 (e.g., if the patient 110 is climbing stairs, etc.). As above, this may affect the significance of any loss of breath experienced by the patient 110. For example, loss of breath while climbing stairs may be less significant than loss of breath experienced while the elevation of the patient is constant. The elevation detection element 122 may be a barometer configured to detect changes in the patient's mode using methods that are known in the art. As above, this may be an element of a laptop computer or mobile telephone used by the patient.

The video recording apparatus 123 records video data of the facial expression of the patient 110 in order to ascertain the well-being (e.g., mood) of the patient, as will be described in further detail below. In one embodiment, the video recording apparatus 123 is a video camera or a group of video cameras, which are disposed in the home of the patient 110, a hospital room housing the patient 110, etc.

The voice recording apparatus 124 records speech of the patient 110 in order to determine whether the patient 110 is out of breath, as will be described in further detail below. In one embodiment, the voice recording apparatus 124 is a microphone, which may be mounted on a hospital bed, on the patient's clothing, etc. In another embodiment, the voice recording apparatus 124 is a built-in microphone or home camera that is part of a mobile telephone used by the patient, an in-home patient monitoring system including microphones and/or video cameras, etc.

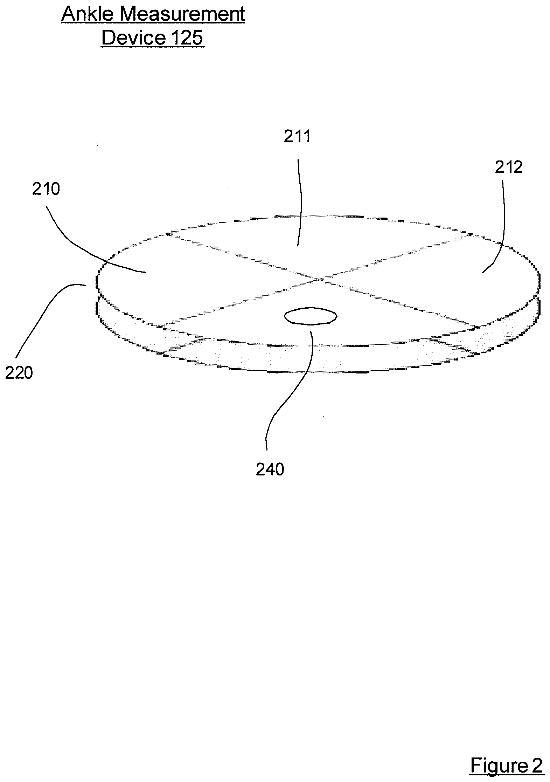

Figure 2 illustrates an exemplary ankle measurement device 125. The ankle measurement device 125 includes a plurality of regions 210, 211 and 212 that correspond to regions of a leg of the patient 110; those of skill in the art will understand that the presence of three regions is only exemplary and that other numbers of regions may be possible in other embodiments. In this exemplary embodiment, the first region 210 corresponds to the patient's ankle, the second region 211 corresponds to a portion of the patient's leg below the knee, and the third region 212 corresponds to a portion of the patient's leg above the knee. The ankle measurement device 125 is constructed in a manner that the patient 110 may press on the various regions 210, 211 and 212 in a manner to simulate pressing on swelling in the corresponding portion of the leg. In one embodiment, an exterior portion 220 of the ankle measurement device 125 is comprised of rubber or a similar elastic material, and the exterior portion 220 encloses a plurality of pressure sensors 230, 231 and 232 (e.g., one pressure sensor corresponding to each of the regions 210, 211 and 212) that respond in a manner that will be described below when the patient 110 presses on the corresponding region. Those of skill in the art will understand that the pressure sensors 230, 231 and 232 are internal to the ankle measurement device 125, and are thus not shown in Figure 2. The ankle measurement device 125 also includes an indicator 240 (e.g., an LED or other light source) that may light up in varying manners to provide feedback to the patient 110, as will be described in further detail below. Those of skill in the art will understand that while the exemplary system 100 includes an ankle measurement device 125, other exemplary systems may include devices adapted to measure pressure in other parts of the body where swelling may indicate edema, such as the abdomen or the hands.

The weight measurement device 126 periodically measures the weight of the patient 110 in order to determine whether weight gain is at a level that may be indicative of a worsening condition, as will be described in further below. The weight measurement device 126 is typically a scale that the patient 100 is instructed to use to weigh himself or herself periodically (e.g., daily, every other day, etc.); in another embodiment, the weight measurement device 126 is embedded into a hospital bed of a patient who is hospitalized.

The system 1 also includes further elements for analyzing the data collected by the elements described above. This may include a mode recognition unit 130, a facial expression recognition unit 131, and a speech recognition unit 132. The mode recognition unit 130 communicates with the position detection element 120, the mode detection element 121, and the elevation detection element 122, as illustrated in Figure 1, and may be, for example, a combination of hardware and software. The mode recognition unit 130 receives data from the above-mentioned elements, and uses the received data to determine a mode of activity for the patient 110. The mode may be, for example, laying, walking, running, climbing stairs, etc.

The facial expression recognition unit 131 may also be a combination of hardware and software, and receives data from the video recording apparatus 123. The facial expression recognition unit 131 monitors the facial expression of the patient 110, and outputs a classification. The classification may be, for example, happy, neutral, unhappy, etc.

The speech recognition unit 132 may also be a combination of hardware and software, and receives data from the voice recording apparatus 124. The speech recognition unit 132 performs sequencing of the recorded speech of the patient 110, and outputs data identifying pauses in speech, length of the pauses, and frequency of the pauses.

The edema evaluation unit 133 may also be a combination of hardware and software, and receives data from the ankle measurement device 125 and the weight measurement device 126. The edema evaluation unit 133 monitors changes in ankle swelling, as measured by the ankle measurement unit 125, and the weight of the patient 110, as measured by the weight measurement unit 126, to determine whether the patient 110 is experiencing edema. As described above, edema may be a sign of worsening heart failure, kidney damage, or COPD.

The system 1 also includes a processing unit 140 receiving data from the mode recognition unit 130, the facial expression recognition unit 131, and the speech recognition unit 132. The data so received is used to make determinations regarding the patient's overall status, as will be described in further detail below. In some embodiments, the processing unit 140 compares the patient's current condition to relevant data that is stored in data storage 150, and the current information about the patient 110 is further stored in the data storage 150 for subsequent use or retrieval. In situations where the patient's symptoms indicate a worsening condition, feedback is provided either to medical professionals 160 (e.g., a doctor, a nurse, etc.), directly to the patient 110, or both as appropriate.

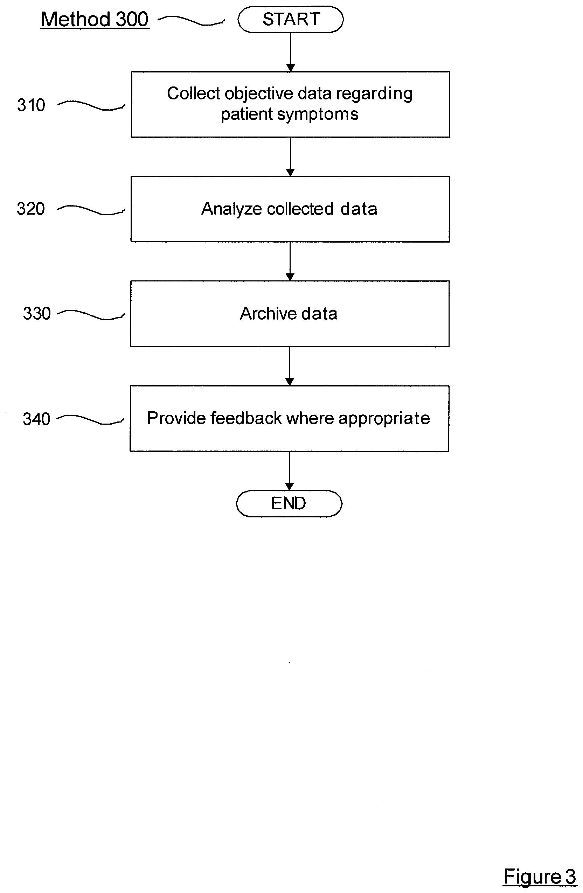

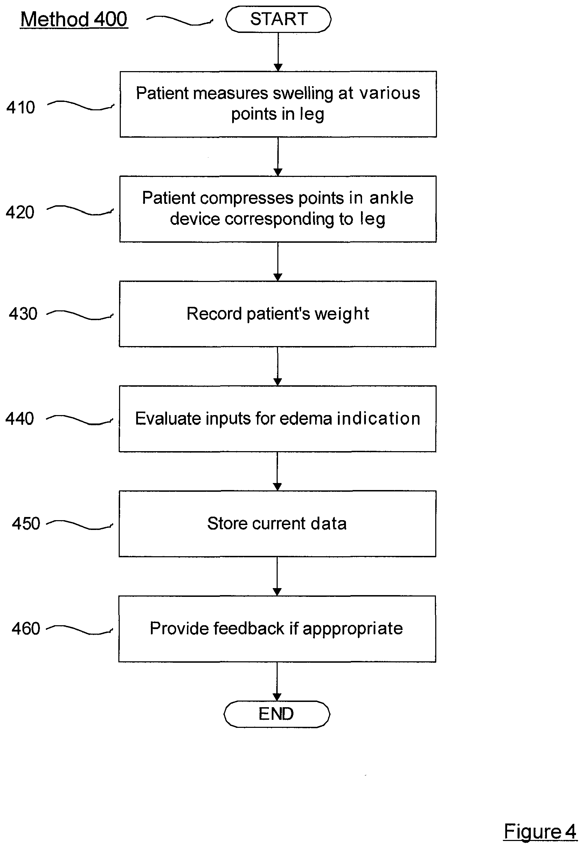

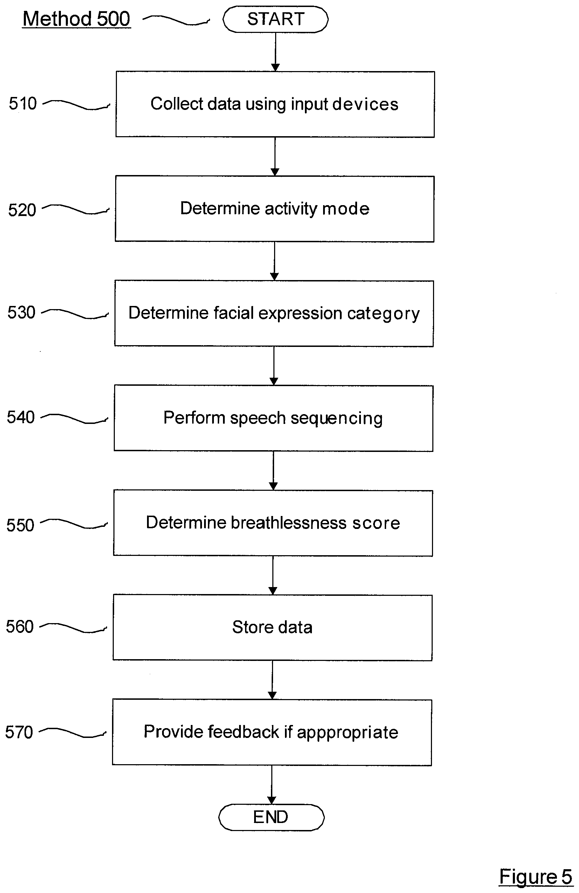

Figure 3 illustrates a generalized exemplary method 300 for collecting and using patient data, which may be implemented using system elements such as those described above with reference to Figure 1. In step 310, objective data regarding symptoms experienced by a patient (e.g., patient 110) is collected. This includes any of the types of data described with reference to the elements of the system 100 of Figure 1, such as data relating to patient position, patient activity, patient elevation, patient facial expression, patient speech, patient weight, patient swelling, etc. In step 320, the data collected in step 310 is analyzed. The analysis step may entail various specific tasks, depending on the nature of patient data that has been collected. Figures 4 and 5, to be discussed below, will illustrate exemplary methods 400 and 500, respectively, detailing specific methods of analyzing specific types of patient data. In step 330, data is archived, such as in data storage 150 of system 100. Data to be archived may include raw data collected in step 310, analyzed data produced in step 320, or both. Last, in step 340, feedback is provided to the patient, to medical professionals, or both.

Figure 4 illustrates a method 400 that is a version of the method 300 that is specific to data collected by the ankle measurement device 125 and the weight measurement device 126 of Figure 1. In step 410, the patient (e.g., patient 110) squeezes his or her leg at various points, each point corresponding to a measurement point on the ankle measurement device 125, to gauge for swelling at various points. As described above, this includes a point at the ankle, a point below the knee, and a point above the knee, although this may vary among differing implementations.

In step 420, the patient squeezes the points on the ankle measurement device 125 corresponding to the points on the leg discussed above (e.g., first region 210 corresponding to a point at the ankle, second region 211 corresponding to a point below the knee, and third region 212 corresponding to a point above the knee, etc.). The ankle measurement device 125 detects and records the pressure exerted by the patient in this step, which corresponds directly to congestion at the point in the leg corresponding to the measurement point in the ankle measurement device 125. Those of skill in the art will understand that the above steps may vary, and that in some embodiments the patient 110 may be instructed to measure at certain points at specified times of the day, at all points each time the device is used, etc., depending on the preferences of the patient's doctor.

In some embodiments, in step 430 the patient's weight is recorded using weight measurement device 126. Thus, it will be apparent to those of skill in the art that steps 410, 420 and 430 are analogous to the data collection step 310 of method 300.

In step 440, the measured data is evaluated, such as by edema evaluation unit 133 of system 100. In one exemplary embodiment, a currently-measured congestion level (e.g., a measurement at a single point on the device, a composite of measurements at several points on the device, etc.) is compared to a baseline congestion level (e.g., the congestion level when the patient 100 was most recently discharged from inpatient care, etc.). A threshold value (e.g., as defined by a doctor) may also be used in this comparison.

In step 450, the currently-measured congestion level or levels are stored, e.g., in data storage 150 of system 100. In step 460, feedback is generated as appropriate. For example, if the current congestion level is acceptable, the indicator 240 on the ankle measurement device 126 is illuminated green to indicate to the patient 100 that the congestion level is acceptable, and no feedback is provided to medical professionals 115. An acceptable level may be defined as a value that is less than the sum of the baseline value and the threshold value. Conversely, if the current congestion level is unacceptable (e.g., indicative of worsening condition), then the indicator 240 on the ankle measurement device 126 is illuminated red to indicate to the patient 100 that the condition may be worsening, and corresponding feedback is provided to medical professionals 115.

In embodiments where weight is measured in step 430, this is also included in the evaluation as described above. In one embodiment, the patient's doctor may determine an acceptable weight range for the patient (e.g., plus or minus two kilograms), and if the patient's weight gain or loss has exceeded the thresholds set by the doctor, that information is considered along with congestion in determining whether the patient 110 is suffering from edema. For example, a weight gain of greater than two kilograms in two days itself trigger an alert as described above. Alternately, the information about the weight gain may be aggregated with the measured congestion, and the aggregated amount is compared to a single threshold value in determining whether to trigger an alert.

Figure 5 illustrates a method 500 that is a version of the method 300 that is specific to data collected by the position detection element 120, the mode detection element 121, the elevation detection element 122, the video recording apparatus 123, and the voice recording apparatus 124 of Figure 1. In step 510, data regarding the patient is collected. This involves collecting data at discrete intervals, aggregating data over a period of time, or continuous collection; those of skill in the art will understand that the method described herein may apply to each of the above methods of collection, and to evaluation on corresponding time scales, without departure from the broader principles described. As will be apparent to those of skill in the art, this includes data recorded by the position detection element 120, the mode detection element 121, the elevation detection element 122, the video recording apparatus 123, and the voice recording apparatus 124 of Figure 1.

In step 520, an activity mode of the patient 110 is determined by the mode recognition unit 130. This may be accomplished using known methods and based on data received from the position detection element 120, the mode detection element 121, and the elevation detection element 122. As described above, the activity mode may be, for example, walking, running, laying, sitting, climbing stairs, standing, etc. Further, the activity mode may be determined continuously, periodically, at discrete intervals, etc., as described above.

In step 530, a facial expression category of the patient 110 is determined by the facial expression recognition unit 131. This may be accomplished using known methods, and may be based on data received from the video recording apparatus 123. In one embodiment, the facial expression may be categorized as "happy", "neutral", or "unhappy". The facial expression is calculated at intervals corresponding to those for which the patient mode is detected in step 520.

In step 540, the speech recognition unit 132 performs speech sequencing on the data provided by the voice recording apparatus 124. This may be accomplished using known methods, and may result in identification of pauses in the speech of the patient 110, the length of the pauses, and their frequency within a given time period. The time period concerned may be a time period corresponding to a time period during which the patient's mode was calculated during step 520, and during which the patient's facial expression was classified during step 530.

In step 550, the processing unit 140 calculates a breathlessness score for the patient 100, based on the results of steps 520, 530 and 540. Each activity mode M may have a corresponding priority rate PW(M), indicating the significance of breathlessness during a particular mode. Activity modes during which the patient is less active may have higher priority rates, indicating that breathlessness during such activity modes is a more serious indication of worsening condition. For example, a mode "laying" has a priority weight 5, a mode "sitting" has a priority weight 4, a mode "walking" has a priority weight 3, a mode "running" has a priority weight 2, and a mode "climbing stairs" has a priority weight 1; those of skill in the art will understand that these priority weights are only exemplary and that the weighting may vary among differing embodiments.

Breathlessness score may be calculated periodically, e.g., once per hour, twice per day, once per day; this may vary among differing embodiments, may be user-configurable (such as by the patient's doctor), etc. During the given interval, the breathlessness score is determined as the sum, over all modes occurring during the time period, of the priority weight of the mode, multiplied by the number of pauses multiplied by the total duration of the pauses during the mode, divided by the duration of the mode. This is expressed as:

$$\sum_M PW(M) \times \frac{\#Pauses \times PausesDuration}{ModeDuration}$$

In other embodiments, the patient facial expression data may also be evaluated as part of the breathlessness score. For example, if breathlessness has been detected and the facial expression also indicates that the patient is unwell, the severity of the breahlessness indication may be increased as a result.

Those of skill in the art will understand that steps 520, 530, 540 and 550 correspond to the analysis step 320 of the method 300 of Figure 3. In step 560, data is stored in the data storage 150. Data to be scored includes raw data, analyzed data (e.g., the breathlessness score) or both. Finally, in step 570, feedback is generated if appropriate. As described above, feedback is provided directly to the patient 110, to medical professionals 115, or both. Feedback may be appropriate, for example, where the current breathlessness score is worse than the score for the previous time interval (e.g., the previous day's breathlessness score), where the breathlessness score is worse than a level specified by a doctor, or based on other criteria that may be appropriate.

The exemplary embodiments thus use objectively recorded data to replace subjective observations or opinions in methods for determining whether the condition of a patient suffering from a chronic illness has worsened. This may provide results that are more reliable, are obtained regularly without subjecting a patient to questionnaires that may yield untrustworthy or missing data, and more accurately predicts the worsening of a patient's condition so that treatment can be sought and administered before an emergency exists.

Those skilled in the art will understand that the above-described exemplary embodiments may be implemented in any number of manners, including, as a separate software module, as a combination of hardware and software, etc. For example, the mode recognition unit 130 may be a program containing lines of code that, when compiled, may be executed on a processor.

It is noted that the claims may include reference signs/numerals in accordance with PCT Rule 6.2(b). However, the present claims should not be considered to be limited to the exemplary embodiments corresponding to the reference signs/numerals.

It will be apparent to those skilled in the art that various modifications may be made in the present invention, without departing from the spirit or the scope of the invention. Thus, it is intended that the present invention cover modifications and variations of this invention provided they come within the scope of the appended claims and their equivalents.

## Claims

1.  A system (100), comprising:

    a voice recording apparatus (124) for recording an audio recording of a patient (110) during a time interval;
    a speech recognition unit (132) for receiving the audio recording and determining, from the audio recording, a number of speech pauses during the time interval and a length of each of the pauses; and
    a processor (140); **characterized in that** the processor (140) is adapted for determining, based on the number of pauses and the length of each of the pauses, a breathlessness score for the patient corresponding to the time interval.

2. The system of claim 1, further comprising:

a position detection apparatus (120) for detecting a position of the patient;
a mode detection apparatus (121) for detecting an activity mode of the patient; and
an elevation detection apparatus (122) for detecting an elevation of the patient,
wherein the breathlessness score is furthermore based on one of the position of the patient, the activity mode of the patient, and the elevation of the patient.

3. The system of claim 1, wherein the breathlessness score is further based on an expression classification of the patient during the time interval.

4. The system of claim 3, further comprising:

a video recording apparatus (123) recording a facial expression of the patient during the time interval,
wherein the expression classification is determined based on the facial expression.

5. A method (500) comprising the steps of:

- receiving (510) an audio recording of a patient during a time interval;
- determining (540), from the audio recording, a number of speech pauses during the time interval and a length of each of the pauses; **characterized in that** the method further comprises the steps of:
- determining (550), based on the number of pauses and the length of each of the pauses, a breathlessness score for the patient corresponding to the time interval; and
- providing (570) an alert to one of the patient and a medical professional, if the determined breathlessness score is greater than an acceptable level.

6. The method according to claim 5, wherein the breathlessness score is further based on a position of the patient detected by a position detection apparatus, an activity mode of the patient detected by a mode detection apparatus, and an elevation of the patient detected by an elevation detection apparatus.

7. The method according to claim 5, wherein the breathlessness score is further based on an expression classification of the patient during the time interval.

8. The method according to claim 7, wherein the expression classification is determined based on a video recording of a facial expression of the patient recorded by a video recording apparatus.

9. Computer program product comprising program code means for causing a computer to carry out the steps of the method as claimed in any of claims 5 to 8 when said computer program is carried out on the computer.

**Patentansprüche**

1. System (100), das Folgendes umfasst:

ein Sprachaufzeichnungsgerät (124) zum Aufzeichnen einer Audioaufzeichnung eines Patienten (110) während eines Zeitintervalls;
eine Spracherkennungseinheit (132) zum Empfangen der Audioaufzeichnung und zum Bestimmen aus der Audioaufzeichnung einer Anzahl von Sprechpausen während des Zeitintervalls und einer Länge jeder der Pausen; und
einen Prozessor (140); **dadurch gekennzeichnet, dass** der Prozessor (140) angepasst ist, um basierend auf der Anzahl von Pausen und der Länge jeder der Pausen eine Atemlosigkeitsnote für den Patienten, die dem Zeitintervall entspricht, zu bestimmen.

2. System nach Anspruch 1, das ferner Folgendes umfasst:

ein Positionserfassungsgerät (120) zum Erfassen einer Position des Patienten;
ein Moduserfassungsgerät (121) zum Erfassen eines Aktivitätsmodus des Patienten; und
ein Höhenlageerfassungsgerät (122) zum Erfassen einer Höhenlage des Patienten,
wobei die Atemlosigkeitsnote ferner auf einer der Position des Patienten, des Aktivitätsmodus des Patienten und der Höhenlage des Patienten basiert.

3. System nach Anspruch 1, wobei die Atemlosigkeitsnote ferner auf einer Ausdruckeinstufung des Patienten während des Zeitintervalls basiert.

4. System nach Anspruch 3, das ferner Folgendes umfasst:

ein Videoaufzeichnungsgerät (123), das einen Gesichtsausdruck des Patienten während des Zeitintervalls aufzeichnet,
wobei die Ausdruckeinstufung basierend auf dem Gesichtsausdruck bestimmt wird.

**5.** Verfahren (500), das folgende Schritte umfasst:

- Empfangen (510) einer Audioaufzeichnung eines Patienten während eines Zeitintervalls;
- Bestimmen (540) aus der Audioaufzeichnung einer Anzahl von Sprechpausen während des Zeitintervalls und einer Länge jeder der Pausen; **dadurch gekennzeichnet, dass** das Verfahren ferner folgende Schritte umfasst:
- Bestimmen (550) basierend auf der Anzahl von Pausen und der Länge jeder der Pausen einer Atemlosigkeitsnote für den Patienten, die dem Zeitintervall entspricht; und
- Bereitstellen (570) einer Warnung zu einem des Patienten und einer medizinischen Fachkraft, falls die bestimmte Atemlosigkeitsnote größer ist als ein akzeptables Niveau.

**6.** Verfahren nach Anspruch 5, wobei die Atemlosigkeitsnote ferner auf einer Position des Patienten, die von einem Positionserfassungsgerät erfasst wird, einem Aktivitätsmodus des Patienten, der von einem Moduserfassungsgerät erfasst wird, und einer Höhenlage des Patienten, die durch ein Höhenlageerfassungsgerät erfasst wird, basiert.

**7.** Verfahren nach Anspruch 5, wobei die Atemlosigkeitsnote ferner auf einer Ausdruckeinstufung des Patienten während des Zeitintervalls basiert.

**8.** Verfahren nach Anspruch 7, wobei die Ausdruckeinstufung basierend auf einer Videoaufzeichnung eines Gesichtsausdrucks des Patienten, die von einem Videoaufzeichnungsgerät aufgezeichnet wird, bestimmt wird.

**9.** Computerprogrammprodukt, das Programmcodemittel umfasst, um einen Computer zu veranlassen, die Schritte des Verfahrens nach einem der Ansprüche 5 bis 8 auszuführen, wenn das Computerprogramm auf dem Computer ausgeführt wird.

**Revendications**

**1.** Système (100) comprenant :

un appareil d'enregistrement vocal (124) pour enregistrer un enregistrement audio d'un patient (110) au cours d'un intervalle de temps ;
une unité de reconnaissance vocale (132) pour recevoir l'enregistrement audio et déterminer à partir de l'enregistrement audio un certain nombre de pauses de la parole au cours de l'intervalle de temps et une longueur de chacune des pauses ; et
un processeur (140) ; **caractérisé en ce que** le processeur (140) est à même de déterminer, sur la base du nombre de pauses et de la longueur de chacune des pauses, un score d'essoufflement pour le patient correspondant à l'intervalle de temps.

**2.** Système selon la revendication 1, comprenant en outre :

un appareil de détection de position (120) pour détecter une position du patient ;
un appareil de détection de mode (121) pour détecter un mode d'activité du patient ; et
un appareil de détection d'élévation(122) pour détecter une élévation du patient,
dans lequel le score d'essoufflement est en outre basé sur l'un(e) de la position du patient, du mode d'activité du patient et de l'élévation du patient.

**3.** Système selon la revendication 1, dans lequel le score d'essoufflement est en outre basé sur une classification d'expression du patient au cours de l'intervalle de temps.

**4.** Système selon la revendication 3, comprenant en outre :

un appareil d'enregistrement vidéo (123) enregistrant une expression faciale du patient au cours de l'intervalle de temps,
dans lequel la classification d'expression est déterminée sur la base de l'expression faciale.

**5.** Procédé (500) comprenant les étapes consistant à :

- recevoir (510) un enregistrement audio d'un patient au cours d'un intervalle de temps ;
- déterminer (540) à partir de l'enregistrement audio un nombre de pauses vocales au cours de l'intervalle de temps et une longueur de chacune des pauses ; **caractérisé en ce que** le procédé comprend en outre les étapes consistant à :
- déterminer (550), sur la base du nombre de pauses et de la longueur de chacune des pauses, un score d'essoufflement pour le patient correspondant à l'intervalle de temps ; et
- fournir (570) une alerte à l'un du patient et d'un professionnel médical si le score d'essoufflement déterminé est supérieur à un niveau acceptable.

**6.** Procédé selon la revendication 5, dans lequel le score d'essoufflement est en outre basé sur une position du patient détectée par un appareil de détection de position, un mode d'activité du patient détecté par un appareil de détection de mode et une élévation du patient détectée par un appareil de détection d'élévation.

7. Procédé selon la revendication 5, dans lequel le score d'essoufflement est en outre basé sur une classification d'expression du patient au cours de l'intervalle de temps.

8. Procédé selon la revendication 7, dans lequel la classification d'expression est déterminée sur la base d'un enregistrement vidéo d'une expression faciale du patient enregistrée par un appareil d'enregistrement vidéo.

9. Produit de programme informatique comprenant des moyens de codage de programme pour amener un ordinateur à effectuer les étapes du procédé selon l'une quelconque des revendications 5 à 8 lorsque ledit programme d'ordinateur est effectué sur l'ordinateur.

Figure 1

System 100

Patient 110

Position Detection Element 120

Mode Detection Element 121

Elevation Detection Element 122

Video Recording Apparatus 123

Voice Recording Apparatus 124

Ankle Measurement Device 125

Weight Measurement Device 126

Mode Recognition Unit 130

Facial Expression Recognition Unit 131

Speech Recognition Unit 132

Edema Evaluation Unit 133

Processing Unit 140

Data Storage 150

Medical Professionals 115

Ankle Measurement
Device 125

211

212

210

220

240

Figure 2

Method 300 — START

310 — Collect objective data regarding patient symptoms

320 — Analyze collected data

330 — Archive data

340 — Provide feedback where appropriate

END

Figure 3

Method 400 ～～ ( START )

410 ～～ | Patient measures swelling at various points in leg |

420 ～～ | Patient compresses points in ankle device corresponding to leg |

430 ～～ | Record patient's weight |

440 ～～ | Evaluate inputs for edema indication |

450 ～～ | Store current data |

460 ～～ | Provide feedback if apppropriate |

( END )

Figure 4

Method 500 ⌒⌒ ( START )

510 ⌒⌒ | Collect data using input devices

520 ⌒⌒ | Determine activity mode

530 ⌒⌒ | Determine facial expression category

540 ⌒⌒ | Perform speech sequencing

550 ⌒⌒ | Determine breathlessness score

560 ⌒⌒ | Store data

570 ⌒⌒ | Provide feedback if apppropriate

( END )

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9833553 A1
- US 20040210159 A1
- US 20100198097 A1
- US 20050137481 A1
- US 20100298649 A1
- WO 2005036525 A1
- US 20050119586 A1